# EUROPEAN PATENT SPECIFICATION

(11) **EP 2 426 047 B1**
(45) Date of publication and mention of the grant of the patent: **02.05.2018**
(21) Application number: 11179884.9
(22) Date of filing: 02.09.2011
(51) Int. Cl.: B63B 35/79, A45F 3/14, A63B 71/08, B65D 81/05, A61F 5/058

(54) **Supporting device**
Stützvorrichtung
Dispositif de support

(30) Priority: 03.09.2010 EP 10425287; 24.02.2011 EP 11155835
(43) Date of publication of application: 07.03.2012
(73) Proprietor: Advance Kites S.r.l., 25057 Sale Marasino (BS) (IT)
(72) Inventor: Mazzucchelli, Alessandro, 25057 Sale Marasino (Brescia) (IT)
(74) Representative: Lunati & Mazzoni S.r.L.

(56) References cited:
- EP-A1- 1 985 265
- EP-A2- 0 836 811
- WO-A1-2010/004270
- DE-A1- 4 344 694
- DE-A1-102004 041 748
- FR-A1- 2 299 874
- US-A- 5 617 650
- US-A1- 2005 137 513

## Description

The present invention relates to a supporting device of the type pointed out in the preamble of the first claim.

In particular, the present invention pertains to a special supporting, protecting or containing device, adapted to receive at least one portion of an object or a person at the inside thereof, so as to protect the same counteracting excessive and localised forces such as those due to shocks or impacts, for example.

Similar devices are provided in patent documents: US-A-2005/137513, US-A-5617650, DE-A-102004041748, EP-A-1985265.

It is known that both in case of transport/storage of objects and in the sports field, one of the most critical aspects is represented by the possibility of damages to objects and persons and therefore the protection devices used during transport/storage or while a physical activity is being practised.

In particular, in the sports field the supporting devices presently used usually have an inner structure adapted to perform almost the whole of the functions for absorption of the aforesaid localised forces, and an outer coating adapted to surround the inner structure avoiding deterioration due to external agents.

Therefore, the most important part is made up of the inner structure that, actually, identifies most of the capacity to absorb localised forces, and therefore the quality of the supporting device.

The inner structure not only performs said function of support or protection but it must also give a high sensation of comfort and offer a functional use and therefore must be made of soft materials so as to have good deformability and therefore ensure good adhesion to the body.

Therefore, the inner structure is made of materials that are strong but at the same time sufficiently soft, such as foam rubber, synthetic polychloroprene rubber, closed- or open-cell foam, polyvinyl chloride.

Some examples of supporting devices thus made can be found in kitesurfing, hang gliding and paragliding.

In particular, in kitesurfing, a supporting device thus made can be identified in the "trapeze", i.e. the supporting band adapted to protect at least the user's back. On the contrary, in hang gliding this device can be identified in the harness, i.e. the structure surrounding the shoulders and almost all the bust. Finally, in paragliding the supporting device can be identified in the so-called "lumbar support", i.e. the device in which the user is housed in a sitting position during the flight and serving as a protection against possible falls.

Similar supporting devices can also be identified in the field of transporting and storing objects, such as bottles, television sets, computers or cameras.

These supporting devices, in a manner similar to the preceding ones, consist of an outer wrapper or shell of polymeric material, adapted to enable support and protection of the product against external agents, and an inner structure adapted to absorb the efforts resulting from a shock or impact. In detail, the inner structure is obtained using polyethylene sheets with air bubbles, of elastomeric material, open-cell or closed-cell solid foams according to the required deformability and resistance features.

The known art mentioned above has some important drawbacks.

In fact, the supporting devices used both for sport activities and for transport/storage of objects, do not have optimal features, i.e. are not able to ensure high safety and comfort/adaptability.

This problem is above all concentrated on the inner structure because said structure must simultaneously meet two substantially opposite requirements, i.e. deformability and hardness or stiffness.

In conclusion, the materials hitherto used for the inner structure have never constituted the ideal choice, but they only represent a compromise between the requirement of appropriate safety (stiffness) and that of comfort and functional use of this sports equipment (deformability).

Another important problem belonging to known supporting devices is given by the reduced deformability and therefore poor adaptability to the profile of the body's portion/object to be protected.

In detail, this poor adaptability causes a non-perfect matching between device and portion to be protected and therefore there are parts of such a portion that, being directly exposed to impacts or other localised forces, are not properly protected by the device.

In conclusion, known supporting devices have a reduced capacity to absorb shocks and therefore are almost useless for storage of brittle products such as bottles or television sets, for example.

A further problem belonging above all to the supporting devices intended for sport activities, is represented by too much thickness of the devices so that the sensation of comfort and the functional use of the devices are reduced.

Under this situation, the technical task underlying the present invention is to conceive a supporting device capable of substantially obviating the mentioned drawbacks.

Within the scope of this technical task, it is an important aim of the invention to create a supporting device capable of absorbing shocks and impacts irrespective of the sizes and shape of the object or the body portion placed inside it.

Another important aim of the invention is to obtain a supporting device characterised by high deformability.

It is a further aim of the invention to obtain a supporting device that, in case of sport activities, ensures a high sensation of comfort.

The technical task mentioned and the aims specified are achieved by a supporting device as claimed in the appended Claim 1.

Preferred embodiments are highlighted in the sub-claims.

The features and advantages of the invention are hereinafter clarified by the detailed description of preferred embodiments of the invention, with reference to the accompanying drawings, in which:
**Fig. 1** shows a supporting device according to the invention;
**Fig. 2a** illustrates a section of the supporting device in Fig. 1 in a first stage of use;
**Fig. 2b** shows the section seen in Fig. 2a in a second stage of use;
**Fig. 2c** shows the section seen in Fig. 2a in a third stage of use;
**Fig. 3** is another supporting device according to the invention;
**Fig. 4a** is a section of the supporting device shown in Fig. 3; and
**Fig. 4b** is a section of the supporting device shown in Fig. 3 in a different stage of use.

With reference to the drawings, the supporting device according to the invention is generally identified with reference numeral **1.**

It is adapted to internally house at least one portion **10** to be supported, protect or contained, belonging to a body or an object, in order to prevent localised forces due to shocks, impacts, external agents such as moisture and temperature changes, from deteriorating said portion 10 or the like.

In particular, the supporting device 1, should the portion to be supported be a part of the body, is used during a physical activity for bearing localised forces or other.

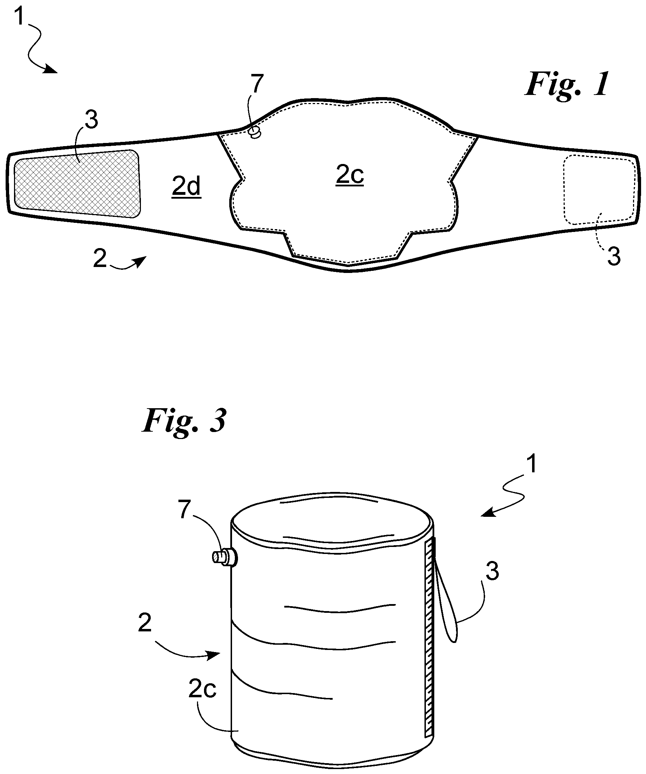

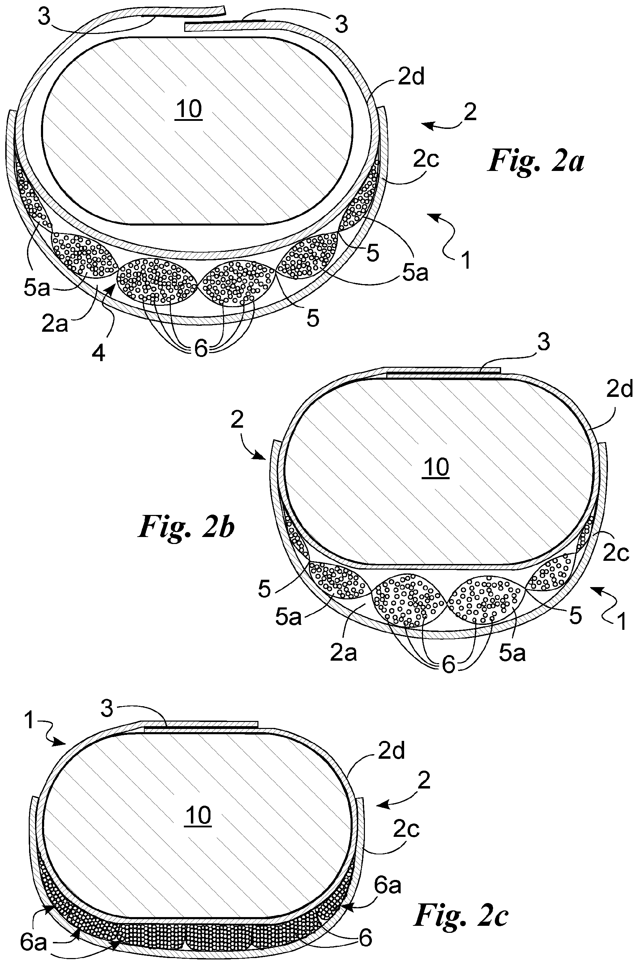

Therefore, the supporting device 1 can consist of an equipment system to be used for increasing safety or the like in performing a physical/sport activity such as kitesurfing, paragliding, hang gliding, or during trips and camping. In particular, it can be a "trapeze" for kitesurfing (Figs. 1 and 2a-2c), a "harness" for hang gliding, a "lumbar support" for paragliding, the backrest or the straps of a camping rucksack, a seat of a sports car, etc.

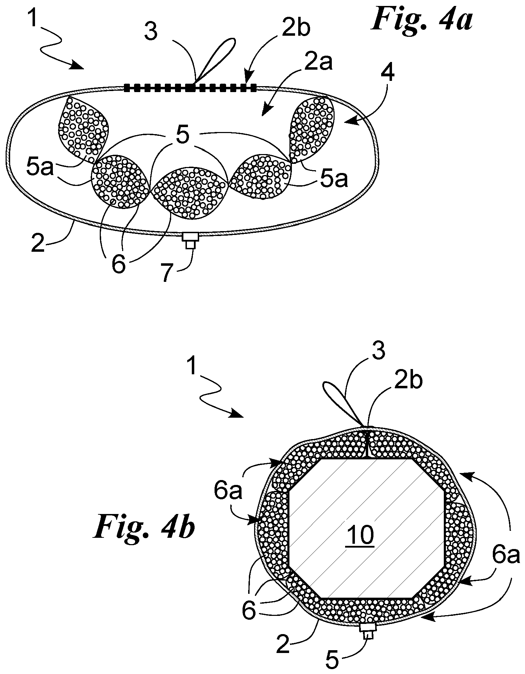

As an alternative to the above described possibilities, the supporting device 1, as shown in Figs. 3 and 4a-4b, can be used for containing and protecting an object and, more specifically, at least one object portion 10 against localised forces and external agents that can deteriorate the object itself during transport or storage of same. In this case, the supporting device 1 can be used for protecting and containing an object of small sizes, such as a bottle or a camera for example, or an object of big sizes such as a computer, a screen or still other objects.

The supporting device 1 comprises an outer wrapper or shell **2** that is impervious to gas passage and adapted to define a gas-tight housing volume **2a;** closing members **3** integral with the outer wrapper 2 and adapted to enable tightening of device 1 on the portion to be protected 10; an inner wrapper **4** permeable to gas passage, defining an operating volume and adapted to be housed inside the housing volume 2a; and separation means **5** adapted to divide said operating volume into a plurality of sub-volumes **5a.**

The outer wrapper 2 is adapted to define a housing volume 2a capable of receiving a portion 10 substantially corresponding to the whole body or object (Figs. 3 and 4a-4b) or, alternatively, corresponding to only part of a body (Figs. 1 and 2a-2c) or object.

In particular, if the portion to be supported 10 is substantially coincident with the whole body or object, the outer wrapper 2 defines a housing volume 2a which is substantially closed and has at least one inlet **2b** (Fig. 4a) adapted to enable portion 10, i.e. the object, to be inserted into and removed from the housing volume 2a. In greater detail, said closing members 3 consist of zips, Velcro (pressure-sensitive adhesive tape fasteners) or other similar means adapted to sealingly shut the inlet 2b preventing air from entering the housing volume 2a and therefore forming an air-tight volume 2a.

Alternatively, if the portion to be supported 10 corresponds to only a limited part of an object or body, the outer wrapper 2 abuts against portion 10 at an edge of the wrapper itself that, in order to form a gas-tight volume 2a, can have elastic bands or other similar means adapted to lock the outer wrapper 2 on portion 10 avoiding passage of gas and various aeriform substances between the housing volume 2a and the environment external thereto. In this case the closing members 3, as better described in the following, close the outer wrapper 2 upon itself locking the supporting device 1 on the portion to be supported 10 (Fig. 2a) and therefore creating said housing volume 2a. To this aim, the closing members 3 can comprise Velcro or other similar means adapted to enable the sizes of the housing volume 2a to be varied so as to cause matching of the outer wrapper 2 with the portion to be protected thereby creating a housing volume 2a of the hermetic type, irrespective of the sizes of portion 10 (Fig. 2b). The outer wrapper 2, in addition to said volume 2a, defines the outer surface of device 1 and therefore is at least partly made of a material resistant to external agents and in particular adapted to inhibit both entry of water or other substances inside volume 2a and deterioration in case of falls or impacts.

In conclusion, the outer wrapper 2 is made of a material impervious to gas, so as to create a sealed volume 2a resistant to said external agents, and in addition preferably elastically deformable so as to enable the protection device 1 to fit the shape of the portion to be protected 10. In detail, it is made of a polymer material and in particular closed-cell EVA foam, neoprene®, polyvinylchloride or other similar material adapted to perform the aforesaid functions.

In addition, in order to ensure high sensation of comfort, the outer wrapper 2 can consist of two mutually linked layers, by means of seams for example, so as to jointly define the housing volume 2a.

In detail, it is possible to identify a coating layer **2c** representing the outer surface of device 1 and made of the aforesaid materials and a resting layer **2d** adapted to come into contact with portion 10 and made of fabric or other similar material suitable to define a comfortable rest surface. In particular, in order to offer a more comfortable use of device 1, the resting coating 2d can have a suitable padding of foam rubber or other similar material.

Inside volume 2a, the protection device 1 has the inner wrapper 4 that preferably is at least partly secured through glue, seams, Velcro or other similar solutions to the outer wrapper 2.

The inner wrapper 4 defines an inner operating volume and is in connection for fluid passage with the housing volume 2a. In particular, the two volumes are in connection for gas or aeriform substance passage so that they have the same pressure. pressure.

This connection for gas passage is obtained due to an inner wrapper 4 provided with small holes or perforations, or preferably an inner wrapper made of a gas-permeable material.

It is also made of an elastically deformable material so that it can vary its length in response to an external load and therefore ensure perfect adhesion to the portion to be supported 10.

In particular, the inner wrapper 4 is made of a material characterised by a low modulus of elasticity. Preferably, it is made of elastane, i.e. a synthetic fibre of polyurethane known on the market with the brand name Lycra®, which is adapted to ensure elasticity as well as the aforesaid connection enabling gas passage between the two volumes.

At the inner volume 4, the supporting device 1 has the separation means 5 adapted to divide the operating volume into a plurality of consecutive sub-volumes 5a.

In particular, means 5 creates sub-volumes 5a substantially characterised by the same elasticity and deformability as the operating wrapper and in connection for gas passage with each other and with the housing volume 2a. In order to maintain elasticity and deformability features and said connection for fluid passage, the separation means 5 consists of partitioning walls made of the same material as that of the operating volume, and therefore elastane, i.e. Lycra®.

The drawings show separation means 5 consisting of seams, which is not in accordance with the invention.

In addition to the above described components, the supporting device 1 comprises a plurality of distinct particles **6** disposed inside the operating volume and, more specifically, sub-volumes 5a, and at least one valve 7, i.e. an apparatus capable of bringing the housing volume 2a and therefore sub-volumes 5a, into communication for gas passage with the environment external to the outer wrapper 1 and therefore to device 1.

In particular, valve 7 is adapted to carry out a connection for gas passage of the type operating upon command, which means that it is able to open and close the connection exclusively based on a command given by the user like the valve used in inflatable mattresses or life belts, for example.

In greater detail, it is a nonreturn valve that inhibits spontaneous entry of air/gas inside the supporting device 1 maintaining the housing volume 2a hermetically sealed and therefore to the desired pressure. More specifically, valve 7 enables Introduction of gas into the housing volume 2a exclusively in response to a given command or action by the user.

Valve 7, enabling air passage between external environment and housing volume 2a and therefore between external environment and sub-volumes 5a, is able to cause a variation in the extension of at least the sub-volumes 5a and to define an expanded configuration and a compressed configuration.

In the expanded configuration (Figs. 2b and 4a), pressure of the housing volume 2a is substantially at least the same as the ambient pressure so that the operating volume, and consequently sub-volumes 5a, substantially have the maximum extension, and therefore the distinct particles 6 are almost free to move inside the sub-volumes 5a.

On the contrary, in the compressed configuration (Figs. 2c and 4b), the housing volume 2a and therefore the different sub-volumes 5a are under negative pressure relative to the external environment and preferably are substantially under vacuum. This condition gives rise to elastic compression of the sub-volumes 5a that reduce their extension and therefore cause mutual compression of the distinct particles 6, so that they become integral with each other forming resistant elements **6a** inside each sub-volume 5a, i.e. a substantially stiff body adapted to protect and support portion 10 to be supported.

To this aim, the distinct particles 6 are made of a material adapted to allow said particles to adhere to each other when compressed. In detail, this material preferably is a polymer and more specifically a thermoplastic polymer.

In addition, in order to ensure the right resistance to each part of the supporting device 1, said device 1 can be internally provided with sub-volumes 5a comprising different amounts of distinct particles 6, so as to obtain resistant elements 6a of different thickness and therefore different capacity to resist impacts or other similar concentrated forces. Preferably, the resistant element 6a has thickness substantially included between 1 cm and 15 cm, and more specifically, substantially included between 2 cm and 6 cm.

Furthermore, in order to always have a resistant element 6a sufficiently thick, and therefore able to conveniently face up to efforts and impacts, the distinct particles 6 take up a portion of the sub-volume 5a, calculated when the supporting device 1 is in the expanded configuration, substantially included between 3% and 50% of said sub-volume 5a . More specifically, this portion is almost included between 10% and 30% of the sub-volume 5a.

Operation of a supporting device 1 described above as regards its structure is the following.

At the beginning the supporting device 1 is in the expanded configuration. i.e. pressure in the housing volume 2a and sub-volumes 5a is substantially equal to the ambient pressure and the sizes of same are almost maximum so that the distinct particles 6 are free to move inside the sub-volumes 5a.

First of all the portion to be protected 10, a camera for example in case of a device 1 fully surrounding the object, or part of an individual's bust in case of a device 1 constituting a "trapeze", is brought into contact with the outer wrapper 2 (Fig. 2a).

At this point, using the closing members 3, device 1 is shut on the portion to be protected (Fig. 2b and 4a) making the outer wrapper 2 define, together with portion 10, a housing volume 2a hermetically sealed, i.e. such arranged that air passage between the housing volume 2a and the external environment is inhibited.

Once the housing volume 2a has been formed, the supporting device 1 is switched from the expanded configuration to the compressed configuration.

In detail, the user, by use of a pump for example, starts causing air downflow through valve 7 from the housing volume 2a to the external environment until the housing volume 2a is substantially under vacuum and the supporting device 1 has reached the compressed configuration (Figs. 2c and 4b).

This action, due to connection for gas passage between the housing volume 2a and sub-volumes 5a, puts the housing volume 2a and sub-volumes 5a under vacuum so that they are elastically compressed and almost perfectly adhere to the portion to be supported 10.

Compression of sub-volumes 5a causes the distinct particles 6 of each sub-volume 5a to compress against each other and therefore become compacted forming a resistant element 6a in each of the sub-volumes 5a into which the operating volume is divided.

Once this operation has been completed, the supporting device 1 has reached the compressed configuration and the user can use the device for carrying/storing an object or practising a sport activity (kitesurfing, paragliding or hang gliding, for example).

When use of the device is over, the supporting device 1 is brought back to the expanded configuration.

In detail, for carrying out this operation, through opening of valve 7 a connection for gas passage is made between the external environment and the housing volume 2a. This connection, due to the under vacuum condition present in the inner volume 2a, makes air come back into the housing volume 2a itself so that the resistant elements 5a are separated.

In fact, entry of air makes the inner pressure of the housing volume 2a to be again almost the same as the ambient pressure and therefore sub-volumes 5a take their maximum extension again leaving the distinct particles 3 free to mutually move thereby loosening the resistant element 6a.

When return to the expanded configuration has been completed, the constraint created by the closing members 3 is loosened and therefore the supporting device 1 is moved away from the portion to be protected 10.

The invention enables important advantages to be achieved.

A first advantage is represented by the high level of support and protection ensured by device 1.

This advantage has been reached due to the innovative division of the operating volume into many sub-volumes 5a therefore ensuring an even distribution of the distinct particles 6 along the whole portion to be supported 10 and therefore creation of resistant elements 6a along all said portion 10.

In fact, arrangement of the distinct particles 6 inside a plurality of sub-volumes 6a avoids accumulation of particles 6 only in some regions of the operating volume; therefore formation of resistant elements 6a only at given regions is prevented and consequently there are no parts of portion 10 that are left without an appropriate protection.

Another important advantage given by the innovative supporting device 1 is represented by the fact that, due to division of the operating volume into several sub-volumes 5a, resistant elements 6a having different thickness can be made. In detail, due to this possibility, resistant elements 6a of greater thickness can be formed at the critical regions (the optical system of a camera, for example) and resistant elements 6a of smaller thickness, that are able to ensure great mobility to the user, can be formed at less critical regions (the camera casing, for example).

In addition, due to the innovative division of the operating volume into several sub-volumes 5a, the supporting device 1 not only is able to ensure high support and protection, but is also characterised by a high sensation of comfort and functional use.

In fact, the possibility of having resistant elements 6a of different thickness allows elements 6a of smaller thickness to be present at the body joints or the like, as compared with those present at more critical points (a hip, for example), so that great freedom of movement is guaranteed to the user without reducing the protection ensured by device 1.

Said high sensation of comfort is further ensured by the fact that, due to use of the distinct particles 6 and division into elastic and deformable sub-volumes 5a, a substantially perfect adhesion is created between the resistant elements 6a and the portion to be protected 10.

This aspect therefore enables the supporting device 1 to be used in practising any physical activity, at the same time ensure high protection and high freedom of movement to the athlete.

Another advantage resulting from the aforesaid almost perfect adhesion between resistant elements 6a and portion to be protected 10 is represented by the fact that device 1 is able to almost perfectly protect any point of portion 10 and therefore ensure a high degree of protection, irrespective of the shape of the portion itself.

A further important advantage obtained by virtue of the device, due to its capacity of both fitting any portion 10 and conveniently protecting every part of said portion 10, resides in that the supporting device 1 is capable of absorbing localised forces of any amount and therefore protecting particularly delicate objects such as television sets, cameras, bottles, computers and others.

Also advantageous is the selection of the manufacturing materials of the outer wrapper 2 enabling the portion to be protected 10 to be isolated from external agents such as water, temperature changes and moisture.

In detail, this aspect together with the high capacity of absorbing impacts and falls belonging to the supporting device 1, enables deterioration of the portion 10 contained therein to be avoided so that an object can be preserved in a very safe manner over a particularly long period of time.

The invention is susceptible of variations falling within the scope of the invention as defined in the appended claims.

## Claims

1. A supporting device (1) comprising an outer wrapper (2) that is impervious to gas passage and is adapted to define a housing volume (2a); an inner wrapper (4) permeable to gas passage, defining an operating volume and adapted to be housed within said housing volume (2a); a plurality of distinct particles (6) disposed inside said operating volume; separation means (5) suitable to divide said operating volume into a plurality of sub-volumes (5a), each of them containing said distinct particles (6); and at least one valve (7) adapted to create, upon command, a connection for fluid passage between said housing volume (2a) and the external environment so as to vary the extension of at least said sub-volumes (5a) and define an expanded configuration in which said distinct particles can freely move inside said sub-volumes (5a), and a compressed configuration in which said distinct particles (6) become compacted defining a plurality of resistant elements (6a); said supporting device (1) being **characterised in that** said separation means (5) consists of partitioning walls made of the same material as that of the operating volume.

2. A supporting device (1) as claimed in claim 1, wherein said separation means (5) is permeable to gas passage.

3. A supporting device (1) as claimed in one or more of the preceding claims, wherein said inner wrapper (4) is elastic.

4. A supporting device (1) as claimed in one or more of claims 2-3, wherein said inner wrapper (4) and separation means (5) are made of elastane.

5. A supporting device (1) as claimed in one or more of the preceding claims, wherein said outer wrapper (2) is deformable.

6. A supporting device (1) as claimed in one or more of the preceding claims, wherein said distinct particles (6) are made of polymeric material.

7. A supporting device (1) as claimed in one or more of the preceding claims, constituting a "trapeze" for kitesurfing.

8. A supporting device (1) as claimed in one or more of claims 1-6, constituting a " lumbar support" for paragliding.

9. A supporting device (1) as claimed in one or more of claims 1-6, constituting a harness for hand gliding.

10. A supporting device (1) as claimed in one or more of claims 1-6, constituting a wrapper adapted to surround an object at least partly.

## Patentansprüche

1. Stützvorrichtung (1) umfassend eine für Gas undurchlässige Außenhülle (2), die geeignet ist, ein Aufnahmevolumen (2a) zu definieren; eine für Gas durchlässige Innenhülle (4), die ein Arbeitsvolumen definiert und geeignet ist, im Inneren des genannten Aufnahmevolumens (2a) untergebracht zu werden; eine Vielzahl im Inneren des genannten Arbeitsvolumens angeordnete getrennte Partikel (6); Trennelemente (5), die geeignet sind, das genannte Arbeitsvolumen in eine Vielzahl von Untervolumen (5a) zu unterteilen, von denen jedes die genannten getrennten Partikel (6) enthält; mindestens ein Ventil (7), das geeignet ist, auf Befehl eine Verbindung für den Flüssigkeitsdurchgang zwischen dem genannten Aufnahmevolumen (2a) und der Außenumgebung zu bilden, um die Ausdehnung der genannten Untervolumen (5a) zu ändern und eine Erweiterungskonfiguration, in der die genannten getrennten Partikel (6) sich frei im Inneren der genannten Untervolumen (5a) bewegen können, und eine Druckkonfiguration, in der die genannten getrennten Partikel (6) zusammengedrückt werden und eine Vielzahl an widerstandsfähigen Elementen (6a) definieren, zu bilden; wobei die genannte Stützvorrichtung (1) **dadurch gekennzeichnet ist, dass** die genannten Trennelemente (5) aus Wänden bestehen und dass die genannten Trennelemente (5) aus dem gleichen Werkstoff wie das Arbeitsvolumen hergestellt sind.

2. Stützvorrichtung (1) nach Anspruch 1, bei der die genannten Trennelemente (5) gasdurchlässig sind.

3. Stützvorrichtung (1) nach einem oder mehreren der vorangegangenen Ansprüche, bei der die genannte Innenhülle (4) elastisch ist.

4. Stützvorrichtung (1) nach einem oder mehreren der Ansprüche 2-3, bei der die genannte Innenhülle (4) und die genannten Trennelemente (5) aus Elastan bestehen.

5. Stützvorrichtung (1) nach einem oder mehreren der vorangegangenen Ansprüche, bei der die genannte Außenhülle (2) verformbar ist.

6. Stützvorrichtung (1) nach einem oder mehreren der vorangegangenen Ansprüche, bei der die genannten getrennten Partikel (6) aus Polymerwerkstoff bestehen.

7. Stützvorrichtung (1) nach einem oder mehreren der vorangegangenen Ansprüche, die ein Trapez für Kitesurfen darstellt.

8. Stützvorrichtung (1) nach einem oder mehreren der vorangegangenen Ansprüche, die einen Sattel zum Gleitschirmfliegen darstellt.

9. Stützvorrichtung (1) nach einem oder mehreren der Ansprüche 1-6, die einen Sicherheitsgurt für Hängeglider darstellt.

10. Stützvorrichtung (1) nach einem oder mehreren der Ansprüche 1-6, die eine zum mindestens partiellen Umhüllen eines Gegenstands geeignete Hülle darstellt.

## Revendications

1. Dispositif de support (1) comprenant une enveloppe extérieure (2) imperméable au passage de gaz et destinée à définir un volume de logement (2a) ; une enveloppe intérieure (4) perméable au passage de gaz, définissant un volume opérationnel et destinée à être logée à l'intérieur dudit volume de logement (2a) ; une pluralité de particules distinctes (6) disposées à l'intérieur dudit volume opérationnel ; des moyens de séparation (5) destinés à diviser ledit volume opérationnel en une pluralité de sous-volumes (5a) chacun desquels contenant lesdites particules distinctes (6) ; et au moins une valve (7) destinée à réaliser sur commande une liaison de passage entre ledit volume de logement (2a) et l'environnement extérieur de manière à varier l'extension d'au moins lesdits sous-volumes (5a) et définir une configuration d'expansion, où lesdites particules distinctes (6) se déplacent librement à l'intérieur desdits sous-volumes (5a), et une configuration de compression où lesdites particules distinctes (6) se compactent pour définir une pluralité d'éléments résistants (6a) ; ledit dispositif de support (1) étant **caractérisé en ce que** lesdits moyens de séparation (5) consistent en des parois et où lesdits moyens de séparation (5) sont réalisés dans le même matériau que le volume opérationnel.

2. Dispositif de support (1) selon la revendication 1, où lesdits moyens de séparation (5) sont perméables au passage de gaz.

3. Dispositif de support (1) selon une ou plusieurs des revendications précédentes, où ladite enveloppe intérieure (4) est élastique.

4. Dispositif de support (1) selon une ou plusieurs des revendications 2-3, où ladite enveloppe intérieure (4) et lesdits moyens de séparation (5) sont en élasthanne.

5. Dispositif de support (1) selon une ou plusieurs des revendications précédentes, où ladite enveloppe extérieure (2) est déformable.

6. Dispositif de support (1) selon une ou plusieurs des revendications précédentes, où lesdites particules distinctes (6) sont en matériau polymère.

7. Dispositif de support (1) selon une ou plusieurs des revendications précédentes, constituant un trapèze pour kitesurf.

8. Dispositif de support (1) selon une ou plusieurs des revendications 1-6, constituant une sellette pour le vol en parapente.

9. Dispositif de support (1) selon une ou plusieurs des revendications 1-6, constituant un harnais pour deltaplane.

10. Dispositif de support (1) selon une ou plusieurs des revendications 1-6, constituant une enveloppe destinée à envelopper au moins en partie un objet.
